# EUROPEAN PATENT SPECIFICATION

(11) **EP 3 411 471 B1**
(45) Date of publication and mention of the grant of the patent: **06.01.2021**
(21) Application number: 17702043.5
(22) Date of filing: 25.01.2017
(51) Int. Cl.: A61P 3/10, C12N 5/071

(54) **IN VITRO PRODUCTION OF PANCREATIC BETA CELLS**
IN-VITRO-HERSTELLUNG PANKREATISCHER BETA-ZELLEN
PRODUCTION IN VITRO DE CELLULES BÊTA PANCRÉATIQUES

(30) Priority: 04.02.2016 EP 16154234
(43) Date of publication of application: 12.12.2018
(73) Proprietor: Société des Produits Nestlé S.A., 1800 Vevey (CH)
(72) Inventor: KRAUS, Marine, 1418 Vuarrens (CH); DIOUM, El Hadji Mamadou, 1162 St-Prex (CH); HALLER, Corinne, 74500 Evian (FR)
(74) Representative: Kamibayashi, Lynne
(86) International application number: PCT/EP2017/051450
(87) International publication number: WO 2017/133931

(56) References cited:
- WO-A2-2013/078376
- ALIREZA REZANIA ET AL: "Reversal of diabetes with insulin-producing cells derived in vitro from human pluripotent stem cells", NATURE BIOTECHNOLOGY, vol. 32, no. 11, 11 September 2014 (2014-09-11), pages 1121-1133, XP055200937, ISSN: 1087-0156, DOI: 10.1038/nbt.3033
- PAGLIUCA FELICIA W ET AL: "Generation of Functional Human Pancreatic [beta] Cells In Vi", CELL, CELL PRESS, US, vol. 159, no. 2, 9 October 2014 (2014-10-09), pages 428-439, XP029073423, ISSN: 0092-8674, DOI: 10.1016/J.CELL.2014.09.040
- E. M. DIOUM ET AL: "A small molecule differentiation inducer increases insulin production by pancreatic cells", PROCEEDINGS OF THE NATIONAL ACADEMY OF SCIENCES, vol. 108, no. 51, 5 December 2011 (2011-12-05), pages 20713-20718, XP055215029, ISSN: 0027-8424, DOI: 10.1073/pnas.1118526109 cited in the application
- SHUIBING CHEN ET AL: "A small molecule that directs differentiation of human ESCs into the pancreatic lineage", NATURE CHEMICAL BIOLOGY, NATURE PUB. GROUP, GB, vol. 5, no. 4, 1 April 2009 (2009-04-01), pages 258-265, XP008164145, ISSN: 1552-4450, DOI: 10.1038/NCHEMBIO.154 [retrieved on 2009-03-15]
- REZANIA ALIREZA ET AL: "Maturation of Human Embryonic Stem Cell-Derived Pancreatic Progenitors Into Functional Islets Capable of Treating Pre-existing Diabetes in Mice", DIABETES, AMERICAN DIABETES ASSOCIATION, US, vol. 61, no. 8, 1 August 2012 (2012-08-01) , pages 2016-2029, XP008162152, ISSN: 0012-1797, DOI: 10.2337/DB11-1711 [retrieved on 2012-06-27]

## Description

### FIELD OF THE INVENTION

The present invention relates to the in vitro production of pancreatic beta cells. More specifically, the invention relates to improved methods for differentiating cells, to insulin-producing cells with properties of mature pancreatic beta cells.

### BACKGROUND TO THE INVENTION

Diabetes mellitus (commonly referred to as diabetes) is a group of metabolic diseases that are characterised by high patient blood sugar levels over a prolonged period. Diabetes affects over 300 million people worldwide.

There are two main types of diabetes (type 1 and type 2), which have different causes and methods of treatment. Type 1 diabetes results from the destruction of the insulin-producing beta cells in the pancreas, commonly through autoimmune mechanisms. Type 2 diabetes results from insulin resistance in peripheral tissues, which is combined with pancreatic beta cell dysfunction.

Management of type 1 diabetes requires regular insulin injections for the lifetime of the patient. In contrast, type 2 diabetes may be controlled by management of diet, weight and physical exercise, however long term treatment may require medication (e.g. metformin or sulphonylureas), potentially combined with insulin injections.

Although patients may be able to manage both type 1 and type 2 diabetes with existing treatments, there are currently no cures and diabetes leads to a significantly increased risk of mortality.

Transplantation of insulin-producing pancreatic beta cells offers promise for the direct treatment of diabetes, in particular type 1 diabetes. Studies have shown that patients transplanted with donor human pancreatic islets can sustain independence from insulin injections for over 5 years (Bellin, M.D. et al. (2012) Am. J. Transplant. 12: 1576-1583). However, such transplantations are limited due to a shortage of donor tissue of sufficient quality.

Access to in vitro-derived insulin-producing pancreatic beta cells, for example those produced from stem cells, may ameliorate the issues holding back transplantation therapy (Rezania et al. (2014) Nat.Biotechnol.32:1121-1133). Furthermore, the resulting uniform supply of beta cells would benefit programs aimed at identifying new pharmaceutical agents for treating the disease. Such screening programs are currently hampered by a lack of suitable cells and high variability in those cells that are available.

Studies have shown that definitive endoderm cells and later pancreatic progenitors can be prepared in vitro with high efficiency and that these cells can differentiate into functional beta cells when transplanted into rodents (Kroon, E. et al. (2008) Nat. Biotechnol. 26: 443-452; Rezania, A. et al. (2012) Diabetes 61: 2016-2029). However, this in vivo differentiation is not well understood and it is unclear whether the process would also occur in humans.

Most attempts at in vitro production of insulin-producing cells from human pancreatic progenitors have given rise to cells with immature or abnormal phenotypes. For example, the resulting cells have failed to provide glucose-stimulated insulin secretion in vitro; lacked appropriate beta cell markers (e.g. NKX6-1 or PDX1); abnormally expressed other hormones (e.g. glucagon); and/or lacked suitable function after transplantation (reviewed in Pagliuca, F.W. et al. (2014) Cell 159: 428-439).

More recent protocols have shown improvements in providing cells that more accurately resemble mature pancreatic beta cells (Pagliuca, F.W. et al. (2014) Cell 159: 428-439). However, distinctions still remain between the cells produced by these methods and naturally-occurring mature beta cells.

Accordingly, there remains a significant need for methods that provide insulin-producing cells with properties more closely aligned with mature pancreatic beta cells.

### SUMMARY OF THE INVENTION

The present inventors have surprisingly found that contacting cells with isoxazole 9 (ISX-9) during an in vitro method of producing insulin-producing beta cells provides cells with a phenotype that more accurately resembles mature pancreatic beta cells compared to the same protocol carried out in the absence of ISX-9. For example, the inventors have found that the resulting cells express MafA in a manner similar to naturally-occurring mature beta cells and secrete insulin in response to glucose stimulation.

ISX-9 has previously been cultured with human cadaveric islets and the MIN6 pancreatic beta cell line (WO2013/078376). In this context, it was found to increase the expression and secretion of insulin in islets that produced low levels of insulin due to prolonged culture (Dioum, E.M. et al. (2011) Proc. Natl. Acad. Sci USA 108: 20713-20718). However, this study was carried out with primary mature beta cells and did not reveal any benefit of using ISX-9 in methods of differentiating beta cell precursors.

In one aspect, the invention provides the use of isoxazole 9 (ISX-9) for producing a beta cell in vitro. The ISX-9 may be used in vitro for producing a beta cell from a precursor cell, such as a pancreatic endoderm (PE) cell, preferably a PE cell.

The ISX-9 may be used to improve the maturation of the beta cell (e.g. during an in vitro method for producing the beta cell from a precursor cell) in comparison to an identical method in the absence of ISX-9 (i.e. used to provide a beta cell which is more mature or more fully differentiated). Maturation of a beta cell may be characterised by the expression of maturing and/or mature beta cell markers, such as MafA, PDX1, NKX6-1, NKX2-2, NeuroD1 and/or insulin, preferably MafA, and/or the ability of the insulin-secreting cell to respond acutely to glucose by processing pro-insulin into insulin and C-peptide and by secreting insulin and C-peptide.

In one embodiment, the beta cell expresses MafA.

In another embodiment, the beta cell additionally expresses PDX1, NKX6-1, NKX2-2, NeuroD1, PCSK1 and/or insulin.

In another aspect, the invention provides the use of isoxazole 9 (ISX-9) for increasing MafA, NKX2-2, NeuroD1 and/or insulin expression in a beta cell in vitro. Preferably, the in vitro use of ISX-9 provides increased expression in a beta cell produced from a precursor cell, such as a stem cell, a pluripotent stem cell or a pancreatic endoderm (PE) cell, preferably a PE cell.

In another aspect, the invention provides an in vitro method for producing a beta cell comprising the step of contacting a cell with isoxazole 9 (ISX-9). The method may be a method of producing a beta cell in vitro from a precursor cell. The cell contacted with ISX-9 is less differentiated than a mature beta cell.

In one embodiment, the cell contacted with ISX-9 is produced in vitro from a a pancreatic precursor cell.

During the method or use of the invention, the ISX-9 may be contacted with a cell that is of a maturity on the continuum between a pancreatic endoderm (PE) cell and a maturing beta cell.

Thus, the cell cultured in the presence of ISX-9 for a particular number of days may be a cell that is of a maturity on the continuum between a pancreatic endoderm (PE) cell and a maturing beta cell. For example, said cell may be cultured for a total of about 1-20 days in the presence of ISX-9.

In one embodiment, the ISX-9 is contacted with a pancreatic endoderm (PE) cell, an immature beta cell and/or a maturing beta cell, preferably a PE cell.

In another embodiment, the cell cultured in the presence of ISX-9 for a particular number of days, such as a total of about 1-20 days, is a PE cell.

It will be understood that the cell will differentiate during the course of the method, so for example the PE cell may have differentiated to a maturing or mature beta cell by the end of the method.

In one embodiment, the method comprises culturing a cell for a total of about 1-20, 1-19, 1-18, 1-17, 1-16, 1-15, 1-14, 1-13, 1-12, 1-11, 1-10, 1-9, 1-8, 1-7, 1-6, 1-5 or 1-4 days in the presence of ISX-9. In another embodiment, the method comprises culturing a cell for a total of about 2-20, 2-19, 2-18, 2-17, 2-16, 2-15, 2-14, 2-13, 2-12, 2-11, 2-10, 2-9, 2-8, 2-7, 2-6, 2-5 or 2-4 days in the presence of ISX-9. In another embodiment, the method comprises culturing a cell for a total of about 3-20, 3-19, 3-18, 3-17, 3-16, 3-15, 3-14, 3-13, 3-12, 3-11, 3-10, 3-9, 3-8, 3-7, 3-6, 3-5 or 3-4 days in the presence of ISX-9. In another embodiment, the method comprises culturing a cell for a total of about 4-20, 4-19, 4-18, 4-17, 4-16, 4-15, 4-14, 4-13, 4-12, 4-11, 4-10, 4-9, 4-8, 4-7, 4-6 or 4-5 days in the presence of ISX-9. In another embodiment, the method comprises culturing a cell for a total of about 4-18, 4-17, 4-16, 4-15 or 4-14 days in the presence of ISX-9. In another embodiment, the method comprises culturing a cell for a total of about 10-18, 10-17, 10-16, 10-15 or 10-14 days in the presence of ISX-9. Preferably, the method comprises culturing a cell for a total of about 4-18 days in the presence of ISX-9.

In one embodiment, the method comprises culturing a cell for a total of about 20, 19, 18, 17, 16, 15, 14, 13, 12, 11, 10, 9, 8, 7, 6, 5, 4, 3, 2 or 1 days in the presence of ISX-9.

The total time of culturing in the presence of ISX-9 may be carried out in one step or may be divided into two or more separate steps, for example three or four steps. Preferably, the total time of culturing in the presence of ISX-9 is divided into two separate steps.

In one embodiment, the method comprises a first step of culturing a cell for about 1-10, 1-9, 1-8, 1-7, 1-6, 1-5, 1-4, 1-3 or 1-2 days, preferably about 1-4 days, in the presence of ISX-9, and a second step of culturing for about 1-15, 1-14, 1-13, 1-12, 1-11, 1-10, 1-9, 1-8, 1-7, 1-6, 1-5, 1-4, 1-3 or 1-2 days, preferably about 1-14 days, in the presence of ISX-9. Preferably, the first and second steps of culturing in the presence of ISX-9 are separated by a step of culturing in the absence of ISX-9. The culturing in the absence of ISX-9 may be, for example, for about 1-10, 1-9, 1-8, 1-7, 1-6, 1-5, 1-4, 1-3 or 1-2 days, preferably about 1-6 days.

In one embodiment, the method comprises a first step of culturing a cell for about 1-7, 2-6 or 3-5 days, preferably about 3-5 days, in the presence of ISX-9, and a second step of culturing for about 8-16, 9-15 or 10-14 days, preferably about 10-14 days, days in the presence of ISX-9. Preferably, the first and second steps of culturing in the presence of ISX-9 are separated by a step of culturing in the absence of ISX-9. The culturing in the absence of ISX-9 may be, for example, for about 2-8, 3-7 or 4-6 days, preferably about 4-6 days.

In another embodiment, the method comprises a first step of culturing a cell for about 10, 9, 8, 7, 6, 5, 4, 3, 2 or 1 days, preferably about 4 days, in the presence of ISX-9, and a second step of culturing for about 15, 14, 13, 12, 11, 10, 9, 8, 7, 6, 5, 4, 3, 2 or 1 days, preferably about 14 days, in the presence of ISX-9. Preferably, the first and second steps of culturing in the presence of ISX-9 are separated by a step of culturing in the absence of ISX-9. The culturing in the absence of ISX-9 may be, for example, for about 10, 9, 8, 7, 6, 5, 4, 3, 2 or 1 days, preferably about 4 days. In a preferred embodiment, the method comprises the steps:
(a) culturing a pancreatic endoderm (PE) cell for about 2-5 days, preferably about 4 days, in the presence of ISX-9;
(b) culturing the cell provided by step (a) for about 4-6 days, preferably about 4 days, in the absence of ISX-9; and
(c) culturing the cell provided by step (b) for about 10-14 days, preferably about 14 days, in the presence of ISX-9.

In another aspect, the invention provides an in vitro-produced beta cell which expresses MafA. The beta cell may be produced in vitro from a precursor cell, such as a pancreatic endoderm (PE) cell.

The beta cell is produced in vitro using isoxazole 9 (ISX-9). The beta cell may be produced from a precursor cell by contacting the precursor cell in vitro with ISX-9. The precursor cell may, for example, be a pancreatic endoderm (PE) cell.

In another aspect, the disclosure provides a beta cell producible in vitro using isoxazole 9 (ISX-9). Preferably, the beta cell expresses MafA. The beta cell may be produced from a precursor cell by contacting the precursor cell in vitro with ISX-9. The precursor cell may, for example, be a pancreatic endoderm (PE) cell. The beta cell may be produced by the method of the invention.

Preferably, the beta cell is a mammalian beta cell, preferably a human beta cell.

In one embodiment, the beta cell is produced in vitro from a pancreatic endoderm (PE) cell.

Preferably, the cell from which the beta cell is produced is a mammalian cell, preferably a human cell.

In one embodiment, the beta cell additionally expresses PDX1, NKX6-1, NKX2-2, NeuroD1 and/or insulin.

In one embodiment, the beta cell provides a glucose-stimulated insulin response (i.e. secretes insulin upon exposure to glucose).

In one embodiment, the beta cell is a maturing beta cell.

In one embodiment, at least 5%, 10%, 20%, 30%, 40%, 50%, 60%, 70%, 80%, 90%, 95%, 96%, 97%, 98%, 99% or 100% of the population of cells express MafA.

In one embodiment, at least 5%, 10%, 15%, 20%, 25%, 30%, 35%, 40%, 45%, 50%, 60%, 70%, 80%, 90%, 95%, 96%, 97%, 98%, 99% or 100%, preferably at least 20%, of the population of cells provide a glucose-stimulated insulin response (i.e. secrete insulin upon exposure to glucose).

### DESCRIPTION OF THE DRAWINGS

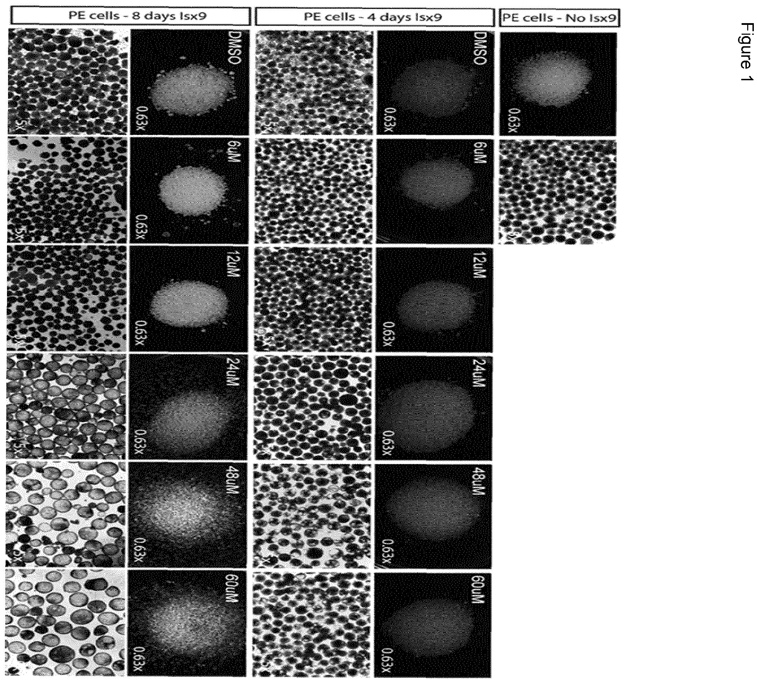

**Figure 1****:** Morphological analysis of PE cells in culture with increasing doses of Isx9. PE cells were cultured in suspension without Isx9 (DMSO control carrier), and with increasing concentrations of Isx9 (6µM, 12µM, 24µM, 48µM and 60µM) for 4 days and 8 days. 5x and 10x magnification pictures have been collected for each condition and time point.

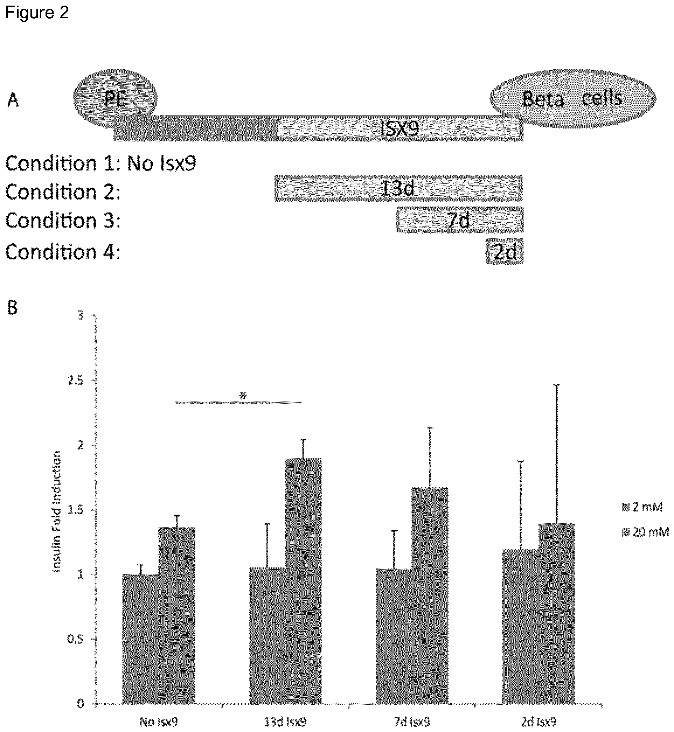

**Figure 2****:** Exposure of PE cells to Isx9 induces glucose response properties to the cells. A: Scheme of the protocol setup. Maturing beta cells have been exposed to DMSO or Isx9 (6µM) for 13 days, 7 days or 2 days during the maturation step. B: In vitro glucose-stimulated Insulin secretion test have been performed to measure secretion of Insulin in each condition (No Isx9, 13 days Isx9, 7 days Isx9 or 2 days Isx9) induced by low glucose concentration (2mM) or high glucose concentration (20mM). The stimulation index of Insulin secretion is determined between the 2 concentrations of glucose on the y axis.

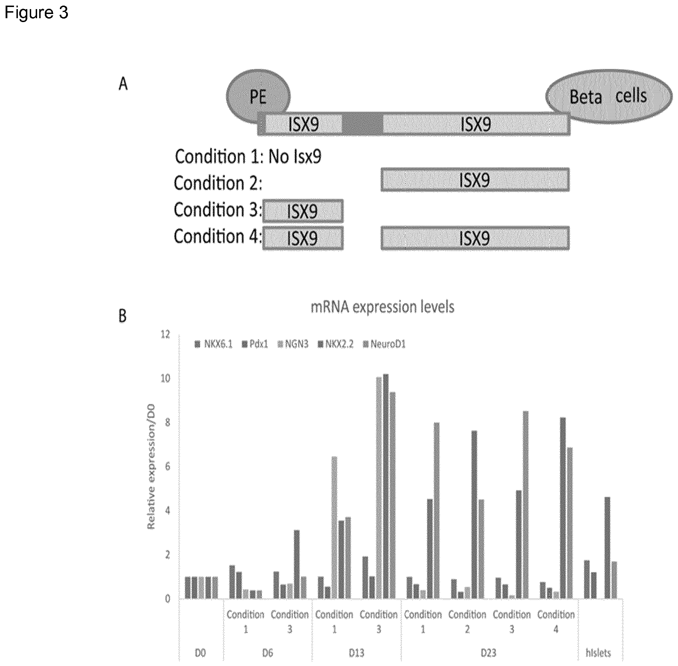

**Figure 3****:** Early exposure of PE cells to Isx9 increases gene expression levels of endocrine progenitors as well as mature beta cell markers. A: Scheme of the protocol setup. B: Early exposure of PE cells to Isx9 (6µM) for 2-6 days (condition 3) increases RNA expression levels of Ngn3, Nkx2.2 and NeuroD1 at D13 of differentiation, as compared to DMSO exposure (condition 1). The increase of Nkx2.2 RNA expression levels is maintained at D23 of differentiation when in vitro maturing beta cells are exposed to Isx9 (6uM) in a two-step protocol (condition 4). Controls are represented by primary human islets. First bar of each pair is 2mM and second bar is at 20 mM.

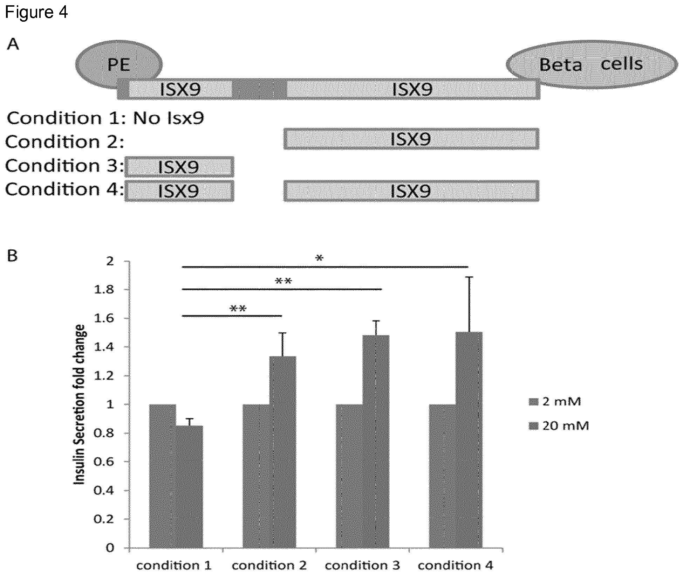

**Figure 4****:** Exposure of PE cells to Isx9 in two-steps increases glucose response properties to the cells. A: Scheme of the protocol setup. In a first step, PE cells have been exposed to DMSO (condition 1) or Isx9 (6µM) for 2-6 days (condition 2). In a second step, maturing beta cells have been exposed to DMSO (condition 3) or Isx9 (6uM) for 10 days (condition 4). B: In vitro glucose-stimulated Insulin secretion test have been performed to measure secretion of Insulin in each condition, induced by low glucose concentration (2mM) or high glucose concentration (20mM). The stimulation index of Insulin secretion is determined between the 2 concentrations of glucose on the y axis. In orderfrom left to right: NKX6.1, Pdx1, NGN3, NKX2-2, NeuroD1.

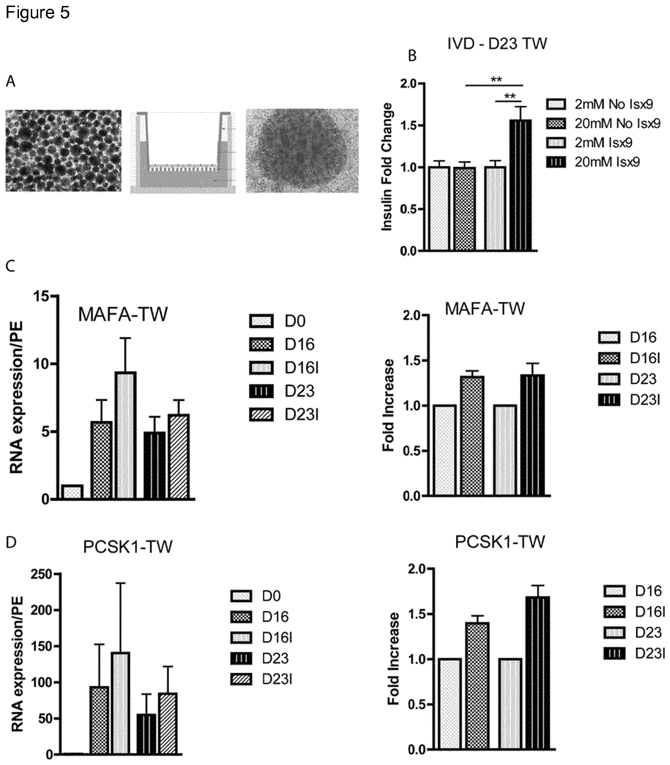

**Figure 5****:** Culturing the PE cells in suspension or in transwell culture systems in presence of Isx9 shows an improved glucose-dependent insulin response

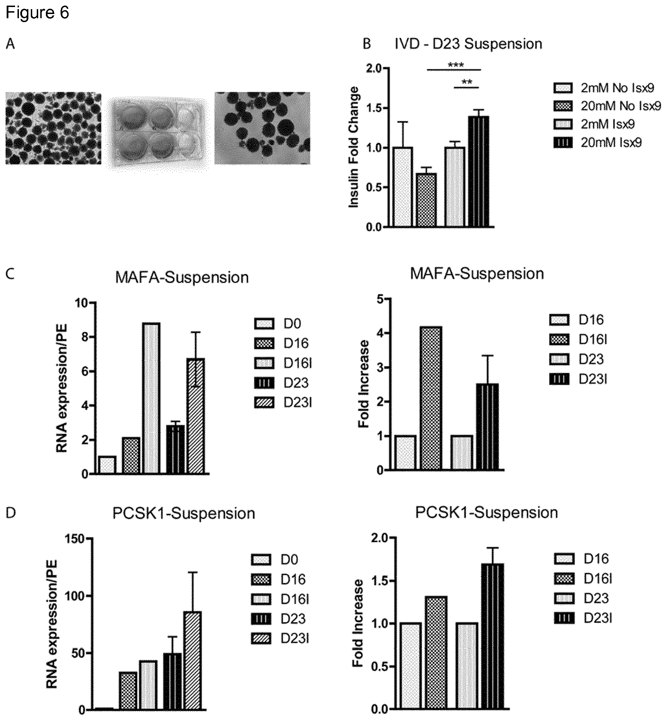

**Figure 6****:** Culturing the PE cells in suspension or in transwell culture systems in presence of Isx9 is showing the same effect of improved glucose-dependent insulin response

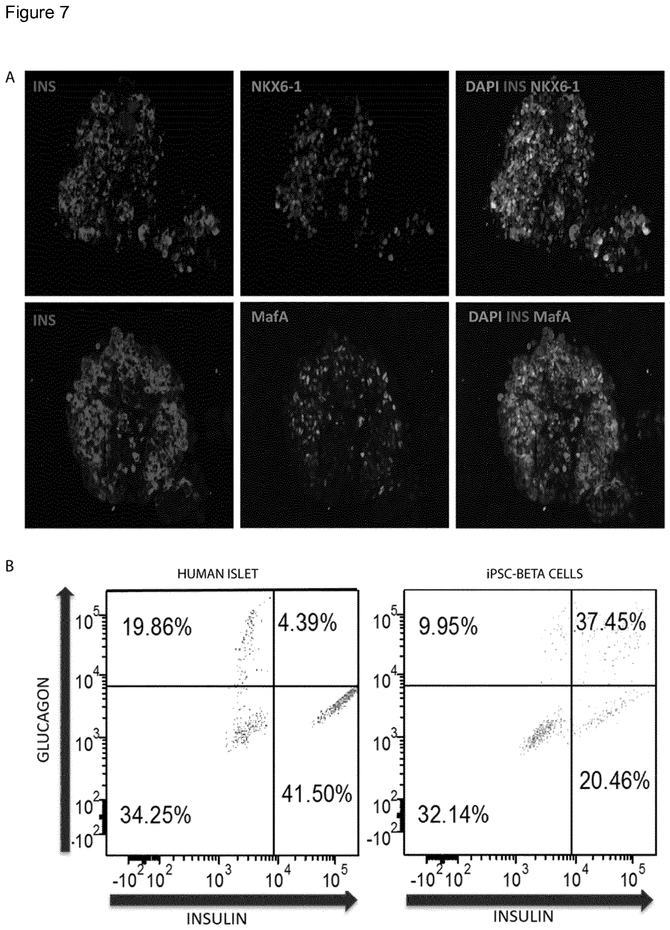

**Figure 7****:** FACS sorting of cells

### DETAILED DESCRIPTION OF THE INVENTION

Various preferred features and embodiments of the present invention will now be described by way of non-limiting examples.

The practice of the present invention will employ, unless otherwise indicated, conventional techniques of chemistry, biochemistry, molecular biology, microbiology and immunology, which are within the capabilities of a person of ordinary skill in the art. Such techniques are explained in the literature. See, for example, Sambrook, J., Fritsch, E.F. and Maniatis, T. (1989) Molecular Cloning: A Laboratory Manual, 2nd Edition, Cold Spring Harbor Laboratory Press; Ausubel, F.M. et al. (1995 and periodic supplements) Current Protocols in Molecular Biology, Ch. 9, 13 and 16, John Wiley & Sons; Roe, B., Crabtree, J. and Kahn, A. (1996) DNA Isolation and Sequencing: Essential Techniques, John Wiley & Sons; Polak, J.M. and McGee, J.O'D. (1990) In Situ Hybridization: Principles and Practice, Oxford University Press; Gait, M.J. (1984) Oligonucleotide Synthesis: A Practical Approach, IRL Press; and Lilley, D.M. and Dahlberg, J.E. (1992) Methods in Enzymology: DNA Structures Part A: Synthesis and Physical Analysis of DNA, Academic Press.

In one aspect, the invention provides the use of isoxazole 9 (ISX-9) for producing a beta cell in vitro.

A "production" of a beta cell refers to production from a less mature precursor cell, for example a stem cell or a cell type on the differentiation pathway between a stem cell and a beta cell (e.g. a pancreatic endoderm (PE) cell, an immature beta cell or a maturing beta cell, preferably a PE cell), by controlled differentiation of that precursor cell. The differentiation to provide the beta cell of the invention may be carried out by culturing the precursor cell under suitable conditions which direct the differentiation of that cell in the desired manner, i.e. towards becoming a maturing or mature beta cell.

### Isoxazole 9 (ISX-9)

Isoxazole 9 (ISX-9; also known as Neuronal Differentiation Inducer III; CAS No. 832115-62-5) is a molecule having the following structure:

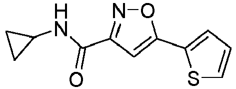

ISX-9 has been shown to induce adult neural stem cell differentiation in vitro and in vivo, and is thought to act through a calcium-activated signalling pathway.

In one embodiment, the ISX-9 is used in the invention at a concentration of about 1-60, 1-50, 1-40, 1-30, 1-20 or 1-10 µM. Preferably, the ISX-9 is used in the invention at a concentration of about 1-10 µM.

In another embodiment, the ISX-9 is used in the invention at a concentration of about 1-11, 2-10, 3-9, 4-8 or 5-7 µM. Preferably, the ISX-9 is used in the invention at a concentration of about 5-7 µM.

In another embodiment, the ISX-9 is used in the invention at a concentration of about 1, 2, 3, 4, 5, 6, 7, 8, 9, 10, 11, 12, 13, 14, 15, 16, 17, 18, 19, 20, 21, 22, 23, 24 or 25 µM. Preferably, the ISX-9 is used in the invention at a concentration of about 6 µM.

### Pancreas

The pancreas is an organ of both the digestive and endocrine systems of vertebrates. It functions as an endocrine gland, producing a number of hormones including insulin, glucagon, somatostatin and pancreatic polypeptide. The pancreas also secretes pancreatic juice containing digestive enzymes into the small intestine.

Beta cells (β cells) are a type of cell naturally found in the pancreatic islets (also known as the islets of Langerhans) of the pancreas. Beta cells typically constitute about 65-80% of the cells in the pancreatic islets.

The main function of beta cells is to store and release insulin, a hormone which causes a reduction in blood glucose concentration once released by the beta cells.

Beta cells are able to rapidly respond to spikes in blood glucose concentration by releasing stored insulin while producing more at the same time.

The uses and methods of the invention enable in vitro production of beta cells from less differentiated cells. The beta cells are cells that are capable of producing insulin, preferably cells that provide a glucose-stimulated insulin response (i.e. secrete insulin upon exposure to glucose).

Beta cells may be characterised, for example, as immature, maturing or mature, in order of development as they become differentiated towards the fully mature beta cells that are naturally found in the pancreas. Preferably, the beta cells of the invention are maturing or mature beta cells.

Immature beta cells may be characterised by the expression of PDX1, NKX6-1, NeuroD1 and Insulin.

Maturing beta cell may be characterised by the expression of PDX1, NKX6-1, NeuroD1, GLUT2, PC1/3, PC2 and Insulin. Preferably, maturing beta cells also express MafA. In another embodiment, the cell capable of producing insulin is responsive to glucose. In another embodiment, the cell capable of producing insulin secretes C-peptide. In another embodiment, the cell capable of producing insulin is a beta-cell.

In the context of the present invention, the term "precursor cell" refers to any cell that may be differentiated to a beta cell when cultured under suitable conditions (e.g. a progenitor cell).

Example precursor cells include stem cells (as described herein), mesendoderm (ME) cells, definitive endoderm (DE) cells, primitive gut (PG) cells, posterior foregut (PF) cells, pancreatic endoderm (PE) cells, pancreatic endocrine precursor cells, immature beta cells and maturing beta cells. Preferably, the precursor cell is a pancreatic endoderm (PE) cell.

ME cells may be characterised by the expression of BRA, FGF4, WNT3 and NCAD.

DE cells may be characterised by the expression of SOX17, CER, FOXA2 and CXCR4.

PG cells may be characterised by the expression of HNF1B and HNF4A.

Posterior foregut (PF) cells may be characterised by the expression of PDX1, HNF6, PROX1 and SOX9.

Pancreatic endoderm (PE) cells may be characterised by the expression of PDX1 and NKX6-1, as well as PTF1A, NGN3 and NKX2-2. PE cells may also include polyhormonal cells expressing insulin and glucagon.

Pancreatic endocrine precursor cells may be characterised by the expression of PDX1, NKX6-1 and NEUROD1.

### MafA

MafA is a transcription factor that specifically binds the insulin enhancer element RIPE3b and activates insulin gene expression. MafA cooperates synergistically with NEUROD1 and PDX1.

MafA is a marker for beta cell maturation.

An example amino acid sequence of human MafA is the sequence deposited under NCBI Accession No. NP_963883.2.

An example amino acid sequence of human MafA is:

### Methods of differentiation

The beta cell of the invention may be produced in vitro from a precursor cell, such as a stem cell and/or a pancreatic endoderm (PE) cell using isoxazole 9 (ISX-9).

In one aspect, the invention provides an in vitro method for producing a beta cell comprising the step of contacting a cell with isoxazole 9 (ISX-9).

The cell contacted with ISX-9 is a precursor cell that has the capacity to differentiate into a beta cell when cultured under suitable conditions.

During the method or use of the invention, the ISX-9 may be contacted with a cell that is of a maturity on the continuum between a pancreatic endoderm (PE) cell and a maturing beta cell.

Thus, the cell cultured in the presence of ISX-9 for a particular number of days may be a cell that is of a maturity on the continuum between a pancreatic endoderm (PE) cell and a maturing beta cell. For example, said cell may be cultured for a total of about 1-20 days in the presence of ISX-9.

In one embodiment, the ISX-9 is contacted with a pancreatic endoderm (PE) cell, an immature beta cell and/or a maturing beta cell, preferably a PE cell.

In another embodiment, the cell cultured in the presence of ISX-9 for a particular number of days, such as a total of about 1-20 days, is a PE cell.

The cell first contacted with ISX-9 may be of a particular type (e.g. a PE cell), however it will be understood that the cell will differentiate during the course of the method, thus ISX-9 may be contacted in a subsequent step with a cell that has differentiated from the cell first contacted with ISX-9 during the course of the method of the invention.

In one embodiment, the method comprises culturing a cell for a total of about 1-20, 1-19, 1-18, 1-17, 1-16, 1-15, 1-14, 1-13, 1-12, 1-11, 1-10, 1-9, 1-8, 1-7, 1-6, 1-5 or 1-4 days in the presence of ISX-9. In another embodiment, the method comprises culturing a cell for a total of about 2-20, 2-19, 2-18, 2-17, 2-16, 2-15, 2-14, 2-13, 2-12, 2-11, 2-10, 2-9, 2-8, 2-7, 2-6, 2-5 or 2-4 days in the presence of ISX-9. In another embodiment, the method comprises culturing a cell for a total of about 3-20, 3-19, 3-18, 3-17, 3-16, 3-15, 3-14, 3-13, 3-12, 3-11, 3-10, 3-9, 3-8, 3-7, 3-6, 3-5 or 3-4 days in the presence of ISX-9. In another embodiment, the method comprises culturing a cell for a total of about 4-20, 4-19, 4-18, 4-17, 4-16, 4-15, 4-14, 4-13, 4-12, 4-11, 4-10, 4-9, 4-8, 4-7, 4-6 or 4-5 days in the presence of ISX-9. In another embodiment, the method comprises culturing a cell for a total of about 4-18, 4-17, 4-16, 4-15 or 4-14 days in the presence of ISX-9. In another embodiment, the method comprises culturing a cell for a total of about 10-18, 10-17, 10-16, 10-15 or 10-14 days in the presence of ISX-9. Preferably, the method comprises culturing a cell for a total of about 4-18 days in the presence of ISX-9.

In one embodiment, the method comprises culturing a cell for a total of about 20, 19, 18, 17, 16, 15, 14, 13, 12, 11, 10, 9, 8, 7, 6, 5, 4, 3, 2 or 1 days in the presence of ISX-9.

The total time of culturing in the presence of ISX-9 may be carried out in one step or may be divided into two or more separate steps, for example three or four steps. Preferably, the total time of culturing in the presence of ISX-9 is divided into two separate steps.

In one embodiment, the method comprises a first step of culturing a cell for about 1-10, 1-9, 1-8, 1-7, 1-6, 1-5, 1-4, 1-3 or 1-2 days, preferably about 1-4 days, in the presence of ISX-9, and a second step of culturing for about 1-15, 1-14, 1-13, 1-12, 1-11, 1-10, 1-9, 1-8, 1-7, 1-6, 1-5, 1-4, 1-3 or 1-2 days, preferably about 1-14 days, days in the presence of ISX-9. Preferably, the first and second steps of culturing in the presence of ISX-9 are separated by a step of culturing in the absence of ISX-9. The culturing in the absence of ISX-9 may be, for example, for about 1-10, 1-9, 1-8, 1-7, 1-6, 1-5, 1-4, 1-3 or 1-2 days, preferably about 1-6 days.

In one embodiment, the method comprises a first step of culturing a cell for about 1-7, 2-6 or 3-5 days, preferably about 3-5 days, in the presence of ISX-9, and a second step of culturing for about 8-16, 9-15 or 10-14 days, preferably about 10-14 days, days in the presence of ISX-9. Preferably, the first and second steps of culturing in the presence of ISX-9 are separated by a step of culturing in the absence of ISX-9. The culturing in the absence of ISX-9 may be, for example, for about 2-8, 3-7 or 4-6 days, preferably about 4-6 days.

In another embodiment, the method comprises a first step of culturing a cell for about 10, 9, 8, 7, 6, 5, 4, 3, 2 or 1 days, preferably about 4 days, in the presence of ISX-9, and a second step of culturing for about 15, 14, 13, 12, 11, 10, 9, 8, 7, 6, 5, 4, 3, 2 or 1 days, preferably about 14 days, days in the presence of ISX-9. Preferably, the first and second steps of culturing in the presence of ISX-9 are separated by a step of culturing in the absence of ISX-

The culturing in the absence of ISX-9 may be, for example, for about 10, 9, 8, 7, 6, 5, 4, 3, 2 or 1 days, preferably about 4 days.

In a preferred embodiment, the method comprises the steps:
(a) culturing a pancreatic endoderm (PE) cell for about 3-5 days, preferably about 4 days, in the presence of ISX-9;
(b) culturing the cell provided by step (a) for about 4-6 days, preferably about 4 days, in the absence of ISX-9; and
(c) culturing the cell provided by step (b) for about 10-14 days, preferably about 14 days, in the presence of ISX-9.

After the step of culturing in the absence of ISX-9 (e.g. step (b)), the cells may have differentiated to become immature beta cells. After the second step of culturing in the presence of ISX-9 (e.g. step (c)), the cells may have differentiated to become maturing or mature beta cells.

In a preferred embodiment, the cell contacted with ISX-9, for example the PE cell.

Methods of the prior art may be used or adapted for the purpose of the invention. For example, methods for culturing PE cells during a process of differentiating to beta cells are described in Pagliuca, F.W. et al. (2014) Cell 159: 428-439. Such methods may be adapted to include cell culture in the presence of ISX-9.

In a particularly preferred embodiment, the in vitro method of the invention comprises the following steps:
(a) culturing a pancreatic endoderm (PE) cell for about 1-3 days, preferably about 2 days, in the absence of ISX-9;
(b) culturing the cell provided by step (a) for about 2-5 days, preferably about 4 days, in the presence of ISX-9;
(c) culturing the cell provided by step (b) for about 4-6 days, preferably about 4 days in the absence of ISX-9;
(d) culturing the cell provided by step (c) for about 10-14 days, preferably about 14 days, in the presence of ISX-9.

Step (a) may be preceded by a further step comprising culturing the PE cell for about 1 day in the presence of DNase I. This is carried out in medium, preferably using a flat bottom plate.

Step (a) may be carried out in S3 BASE medium (MCDB131, 8 mM _{D}-glucose, 1.23 g/L NaHCO₃, 2% FFA BSA, 1:200 ITX-X, 2 mM glutamax, 10 U/ml of penicillin and 10 ug/ml of streptomycin and 0.25 mM vitamin C). Preferably, this medium further comprises 50 ng/mL KGF, 0.25 µM Sant1, 100nM RA and 10 µM Y-27632 (Rock Inhibitor).

Step (b) may be carried out in S5 BASE medium (MCDB131, 20 mM _{D}-glucose, 1.754 g/L NaHCO₃, 2% FFA BSA, 1:200 ITX-X, 2 mM glutamax, 10 U/ml of penicillin and 10 ug/ml of streptomycin , 0.25 mM vitamin C and 10 µg/mL heparin). Preferably, this medium further comprises 100 nM RA, 1 µM XXI, 0.25 µM Sant1, 10 µM Alk5i II, 1 µM T3, 20 ng/mL beta-cellulin and 10 µM Y-27632 (Rock Inhibitor).

The first 3 days of step (c) may be carried out in S5 BASE medium. Preferably, this medium further comprises 25 nM RA, 1 µM XXI, 10 µM Alk5i II, 1 µM T3, 20 ng/mL betacellulin and 10 µM Y-27632 (Rock Inhibitor).

The final 1 day of step (c) may be carried out in maturing BASE medium (CMRL 1066, 10% FBS and 10 U/ml of penicillin and 10 ug/ml of streptomycin Preferably, this medium further comprises 10 µM Alk5i II, 1 µM T3 and 10 µM Y-27632 (Rock Inhibitor).

Step (d) may be carried out in maturing BASE medium. Preferably, this medium further comprises 10 µM Alk5i II, 1 µM T3 and 10 µM Y-27632 (Rock Inhibitor).

Preferably, the ISX-9 is used at a concentration of about 6 µM.

Preferably, steps (b)-(d) are carried out using air-liquid interface culture.

The methods of the invention may comprise contacting a precursor cell with isoxazole 9 (ISX-9), wherein the precursor cell has itself been produced in vitro by the differentiation of a stem cell.

Thus, the methods of the disclosure may comprise the step of producing a cell in vitro from a stem cell.

Preferably, the stem cells of the invention are pluripotent stem cells.

Pluripotent stem cells are stem cells that may propagate indefinitely and differentiate into all cell types of the human body. These stem cells hold promise in providing a single source of cells that may replace cells affected by damage or disease.

Pluripotent stem cells may be created through a number of techniques, such as the generation of induced pluripotent stem cells or embryonic stem cells.

Preferably, the stem cells of the disclosure are induced pluripotent stem cells (iPSCs).

iPSCs are a type of pluripotent stem cell that may be created directly from adult cells. The skilled person is readily able to prepare iPSCs, for example by introducing specific transcription factors into adult cells or contacting adult cells with specific protein combinations.

iPSCs are advantageous over embryonic stem cells in that they overcome the need for using embryonic material and can be prepared from a subject to which they (or cells produced from them) are later re-introduced. Such autologous cell transplantation may overcome the risk of immune rejection of transplanted material.

Methods are known in the art for producing pluripotent stem cells, such as mammalian embryonic stem cells, without the destruction of an embryo. In particular, it has been shown that mouse and human embryonic stem cells may be produced from single blastomeres while leaving the embryo intact. For reference only, Chung, Y. et al. (2006) Nature 439: 216-219 describes methods for making mouse embryonic stem cells from a single blastomere. Later advances on this procedure provided methods where co-culturing the blastomere cell lines with other ESCs is not required (Chung, Y. et al. (2008) Cell Stem Cell 2: 113-117).

Preferably the stem cell of the disclosure is a mammalian stem cell, preferably a human stem cell.

### Diseases

The beta cells may be used in the treatment or prevention of diseases characterised by high blood sugar levels over a prolonged period. Such conditions include insulin resistance; prediabetes; type 1 or 2 diabetes; and metabolic syndrome.

Insulin resistance is a condition in which the body produces insulin but does not use it effectively. When subjects have insulin resistance, glucose builds up in the blood instead of being absorbed by the cells, leading to type 2 diabetes or prediabetes. Insulin resistance may be defined as a reduced responsiveness of a target cell or a whole organism to the insulin concentration to which it is exposed. This definition is generally used to refer to impaired sensitivity to insulin-mediated glucose disposal.

Prediabetes is the medical stage in which not all of the symptoms required to diagnose a person as diabetic are present, but blood sugar is abnormally high. Prediabetes usually occurs in subjects who already have insulin resistance. Although insulin resistance alone does not cause type 2 diabetes, it often sets the stage for the disease by placing a high demand on the insulin-producing beta cells. In prediabetes, the beta cells can no longer produce enough insulin to overcome insulin resistance, causing blood glucose levels to rise above the normal range.

Diabetes mellitus (commonly referred to as diabetes) is a group of metabolic diseases that are characterised by high patient blood sugar levels over a prolonged period. The two main types of diabetes (type 1 and type 2) have different causes and methods of treatment.

Type 1 diabetes results from the destruction of the insulin-producing beta cells in the pancreas, commonly through autoimmune mechanisms.

Type 2 diabetes results from insulin resistance in peripheral tissues, which may be combined with pancreatic beta cell dysfunction.

Type 2 diabetes is a chronic metabolic disorder which is increasing in prevalence globally. In some countries of the world the number of subjects affected is expected to double in the next decade due to an increase in the ageing population.

Type 2 diabetes is characterised by insulin insensitivity as a result of insulin resistance, declining insulin production and eventual pancreatic beta-cell failure. This leads to a decrease in glucose transport to the liver, muscle cells and fat cells. There is an increase in the breakdown of fat associated with hyperglycaemia.

As a result of this dysfunction, glucagon and hepatic glucose levels that rise during fasting are not suppressed with a meal. Given inadequate levels of insulin and increased insulin resistance, hyperglycaemia results.

Subjects with type 2 diabetes are more vulnerable to various short- and long-term complications, including diabetic ketoacidosis (DKA), hyperosmolar hyperglycaemic state (HHS), retinopathy, cardiopathy, nephropathy and neuropathy. These complications may lead to premature death.

Metabolic syndrome is a clustering of at least three of five of the following medical conditions: abdominal (central) obesity, elevated blood pressure, elevated fasting plasma glucose, high serum triglycerides and low high-density lipoprotein (HDL) levels. Metabolic syndrome is associated with the risk of developing cardiovascular disease and diabetes.

### Method of treatment

In one aspect, the disclosure provides the beta cell of the disclosure for use in therapy, for example in treating or preventing diabetes.

The treatment or prevention may comprise transplantation of the beta cell of the invention to a subject in need thereof. Thus, the disclosure provides a method of treating or preventing diabetes comprising the step of transplanting the beta cell of the disclosure to a subject in need thereof.

The diabetes to be treated or prevented may, for example, be type 1 or type 2 diabetes. Preferably the diabetes to be treated or prevented is type 1 diabetes.

### Transplantation

The beta cells may, for example, be administered to a subject as part of an autologous cell transplant procedure or as part of an allogeneic cell transplant procedure. The term "autologous cell transplant procedure" refers to a procedure in which the precursor cells (from which the beta cells are produced) are obtained from the same subject as that to which the beta cells are administered.

Autologous transplant procedures are advantageous as they avoid problems associated with immunological incompatibility and are accessible to subjects irrespective of the availability of a genetically matched donor.

The term "allogeneic cell transplant procedure" refers to a procedure in which the precursor cells (from which the beta cells are produced) are obtained from a different subject as that to which the beta cells are administered. Preferably, the donor will be genetically matched to the subject to which the beta cells are administered to minimise the risk of immunological incompatibility.

Suitable doses of the beta cells are such as to be therapeutically and/or prophylactically effective. The dose to be administered may depend on the subject and condition to be treated, and may be readily determined by a skilled person.

### Pharmaceutical composition

The cells may be formulated for administration to subjects with a pharmaceutically acceptable carrier, diluent or excipient. Suitable carriers and diluents include isotonic saline solutions, for example phosphate-buffered saline, and potentially contain human serum albumin.

### EXAMPLES

### Example 1

### Materials and methods

Frozen PE cells were thawed and cultured for 1 day in I medium containing 10 U/mL DNase I in ULA 6WP flat-bottomed plates.

The PE cells were subsequently transferred to S3 BASE medium (MCDB131, 8 mM _{D}-glucose, 1.23 g/L NaHCO₃, 2% FFA BSA, 1:200 ITX-X, 2 mM glutamax, 100× PS and 0.25 mM vitamin C) containing 50 ng/mL KGF, 0.25 µM Sant1, 100nM RA and 10 µM Y and cultured for a further 2 days.

The resulting cells were then cultured for 4 days using an air-liquid interface culture protocol in Transwell plates. The culture was carried out in S5 BASE medium (MCDB131, 20 mM _{D}-glucose, 1.754 g/L NaHCO₃, 2% FFA BSA, 1:200 ITX-X, 2 mM glutamax, 100× PS, 0.25 mM vitamin C and 10 µg/mL heparin) containing 100 nM RA, 1 µM XXI, 0.25 µM Sant1, 10 µM Alk5i II, 1 µM T3, 20 ng/mL beta-cellulin, 10 µM Y and 6 µM isoxazole 9 (ISX-9).

Subsequently, the medium was exchanged for S5 BASE medium containing 25 nM RA, 1 µM XXI, 10 µM Alk5i II, 1 µM T3, 20 ng/mL beta-cellulin and 10 µM Y and the cells were cultured for a further 3 days. ISX-9 was not present in this medium.

Finally, the medium was exchanged for S6 BASE medium (CMRL 1066, 10% FBS and 100× PS) containing 10 µM Alk5i II, 1 µM T3 and 10 µM Y and the cells were cultured for a further 15 days. ISX-9 was added to this medium at a concentration of 6 µM for the final 14 days of culture (i.e. the first day of culture in this step was carried out in the absence of ISX-9).

### Example 2

Dose response tests have been performed to determine toxicity of Isx9 and the optimal concentration of Isx9 to be tested for beta cell differentiation *in vitro.* High concentrations of Isx9 (from 24µM up to 60µM) appears to be toxic as the aggregates of PE cells become cystic after 8 days in culture as shown in Figure 1. In contrast, the morphology of the aggregates of PE cells is unchanged as compared to the control DMSO condition when using 6 and 12µM of Isx9. In order to maintain physiological condition, a concentration of 6µM of Isx9 has been used for the entire protocol. Exposure of maturing beta cells to Isx9 for 13 days is increasing the glucose-stimulated insulin secretion of the cells as compared to the control DMSO condition (Figure 2). This effect is correlated with the duration of exposure to Isx9 at 6uM. This demonstrates that Isx9 stimulates maturation of iPSC-derived maturing beta cells. The effect of Isx9 is reinforced when adding Isx9 in a first-step at the stage of PE cells as shown in Figure 3 and Figure 4. Isx9 induces an increase in RNA expression levels of endocrine markers such as Ngn3, but also beta cell markers as NeuroD1 and Nkx2.2. These results demonstrate that Isx9 is not only improving the function of the in vitro generated beta cells but ameliorates the maturation state by inducing key beta cell specific transcription factors.

Moreover, culturing the PE cells in suspension or in transwell culture systems in presence of Isx9 is showing the same effect of improved glucose-dependent insulin response as shown in Figure 5 and Figure 6. Interestingly, Isx9 is inducing an increase of MAFA gene expression level as well as PCSK1. Both markers are critical for mediating the response of insulin secretion mediated by glucose. The expression of MAFA can also be unprecedently detected by immunohistochemistry as early as at D9 of differentiation as shown in Figure 7. However, the analysis of the cell composition of the maturing beta cells does not receal an increase in the number or quantity of insulin positive cells when adding Isx9 as compared to DMSO which suggests that Isx9 is promoting maturation of the iPSC-derived beta cells but not increasing the cell fraction.

All the results confirm that Isx9 improves maturation of iPSC-derived PE cells towards beta cells in 3D culture systems by promoting MAFA expression.

Various modifications and variations of the described cells, uses, and methods of the present invention will be apparent to those skilled in the art.

Although the present invention has been described in connection with specific preferred embodiments, it should be understood that the invention as claimed should not be unduly limited to such specific embodiments.

## Claims

1. Use of isoxazole 9 (ISX 9) for producing a beta cell in vitro, wherein the beta cell is produced in vitro from a pancreatic endoderm (PE) cell.

2. The use of claim 1, wherein the beta cell expresses MafA, and preferably additionally expresses PDX1, NKX6-1, NKX2-2, NeuroD1 and/or insulin.

3. An in vitro method for producing a beta cell wherein the cell contacted with isoxazole 9 (ISX-9) is a pancreatic endoderm (PE) cell, an immature beta cell or a maturing beta cell.

4. The method of claim 3, wherein the cell contacted with ISX-9 is a pancreatic endoderm (PE) cell.

5. The method of claim 3 or 4, wherein the method comprises the steps:
(a) culturing a pancreatic endoderm (PE) cell for 2-5 days, preferably 4 days, in the presence of ISX-9;
(b) culturing the cell provided by step (a) for 4-6 days, preferably 4 days, in the absence of ISX-9; and
(c) culturing the cell provided by step (b) for 10-14 days, preferably 14 days, in the presence of ISX-9.

## Patentansprüche

1. Verwendung von Isoxazol 9 (ISX 9) zur Herstellung einer Beta-Zelle in vitro, wobei die Beta-Zelle in vitro aus einer Zelle des pankreatischen Entoderms (PE) hergestellt wird.

2. Verwendung nach Anspruch 1, wobei die Beta-Zelle MafA exprimiert und vorzugsweise zusätzlich PDX1, NKX6-1, NKX2-2, NeuroD1 und/oder Insulin exprimiert.

3. In-vitro-Verfahren zur Herstellung einer Beta-Zelle, wobei die mit Isoxazol 9 (ISX-9) in Kontakt gebrachte Zelle eine Zelle des pankreatischen Entoderms (PE), eine unreife Beta-Zelle oder eine reifende Beta-Zelle ist.

4. Verfahren nach Anspruch 3, wobei die mit ISX-9 in Kontakt gebrachte Zelle eine Zelle des pankreatischen Entoderms (PE) ist.

5. Verfahren nach Anspruch 3 oder 4, wobei das Verfahren die folgenden Schritte umfasst:
(a) Kultivieren einer Zelle des pankreatischen Entoderms (PE) für 2-5 Tage, vorzugsweise 4 Tage, in Gegenwart von ISX-9;
(b) Kultivieren der durch Schritt (a) bereitgestellten Zelle für 4-6 Tage, vorzugsweise 4 Tage, in Abwesenheit von ISX-9; und
(c) Kultivieren der durch Schritt (b) bereitgestellten Zelle für 10-14 Tage, vorzugsweise 14 Tage, in Gegenwart von ISX-9.

## Revendications

1. Utilisation d'isoxazole 9 (ISX 9) pour produire une cellule bêta in vitro, dans laquelle la cellule bêta est produite in vitro à partir d'une cellule d'endoderme pancréatique (PE).

2. Utilisation selon la revendication 1, dans laquelle la cellule bêta exprime MafA, et exprime en outre PDX1, NKX6-1, NKX2-2, NeuroD1 et/ou l'insuline.

3. Procédé in vitro pour produire une cellule bêta dans lequel la cellule mise en contact avec de l'isoxazole 9 (ISX-9) est une cellule d'endoderme pancréatique (PE), une cellule bêta immature ou une cellule bêta en cours de maturation.

4. Procédé selon la revendication 3, dans lequel la cellule mise en contact avec ISX-9 est une cellule d'endoderme pancréatique (PE).

5. Procédé selon la revendication 3 ou 4, dans lequel le procédé comprend les étapes :
(a) culture d'une cellule d'endoderme pancréatique (PE) pendant 2 à 5 jours, de préférence 4 jours, en présence d'ISX-9 ;
(b) culture de la cellule fournie par l'étape (a) pendant 4 à 6 jours, de préférence 4 jours, en l'absence d'ISX-9 ; et
(c) culture de la cellule fournie par l'étape (b) pendant 10 à 14 jours, de préférence 14 jours, en présence d'ISX-9.


### SEQUENCE LISTING

<110> Nestec S.A
<120> Case 15156
<160> 1
<170> BiSSAP 1.2
<210> 1
   <211> 353
   <212> PRT
   <213> Homo sapiens
<400> 1